# EUROPEAN PATENT APPLICATION

(11) **EP 2 535 074 A2**
(43) Date of publication of application: **19.12.2012**
(21) Application number: 12171732.6
(22) Date of filing: 13.06.2012
(51) Int. Cl.: A61M 5/315

(54) **Injector and device for detecting injection button**

(30) Priority: 14.06.2011 KR 20110057696
(71) Applicant: Samsung Electronics Co., Ltd., Suwon-si, Gyeonggi-do, 443-742 (KR)
(72) Inventor: Oh, Jung-Taek, 443-742 Gyeonggi-do (KR)
(74) Representative: Jenkins, Richard Gavin

(57) **Abstract**

An injector (100) includes an injection button (111); and a coil (110) for radiating energy that informs an injection button detection device (200) combined with the injector, of an input of the injection button. The injection button detection device (200) includes a transmitter for radiating an electromagnetic wave associated with a specific voltage to radiate energy that informs an injector combined with the injection button detection device, of an input of an injection button; a receiver for receiving a voltage based on energy radiated from the injector; and a controller for detecting an input of the injection button based on the voltage received from the receiver.

## Description

### BACKGROUND OF THE INVENTION

### 1. Field of the Invention

The present invention relates generally to an injector and a device for detecting an injection button and, more particularly, to an injector and injection button detection device capable of detecting an input of an injection button of the injector, with which the injection button detection device is combined, without direct electrical or mechanical contact.

### 2. Description of the Related Art

Insulin is essential for survival. Among diabetics, a Type-1 patient must continuously measure their blood sugar (glucose) level and inject an adequate amount of insulin based on the measurement. Insulin pens allow individuals to conveniently inject themselves with insulin. Insulin pens also allow individuals to determine a dosage amount by turning a knob, on which possible dosages are written. Insulin is injected by pressing a button mounted on an end of the insulin pen.

Currently, a patient must record by himself information about when and the amount of insulin that has been injected. Based on this self-recorded information, a doctor can check if the patient's disease is under control, or if the treatment must be varied to increase effectiveness.

Only complex systems are presently available to measure glucose levels on a real time basis, i.e. 24 hours a day, and to continuously inject a small amount of insulin as needed, for example by an insulin pump. In contrast, use of the insulin pen requires an individual to periodically check their glucose level, to make required adjustments, and to inject an adequate amount of insulin by themselves. There is presently no low-cost method for determining when and how much insulin an individual has injected, or to transmit the injection results for future use.

To address these and other problems, a dosage measurement device for an injector has been provided, for example as disclosed in Korean Patent Application No. 10-2010-0070547, to measure dosage without modification of the mechanical structure.

However, to use an insulin pen to inject insulin, a patient must first input (push) an injection button of the insulin pen to initiate the insulin injection.

However, when the dosage measurement device is detachable from the insulin pen, the dosage measurement device may not accurately detect an input of the injection button of the insulin pen. Accordingly, efforts by the patient to record information about insulin self-injections are largely ineffective.

In addition, for a disposable insulin pen, a dosage measurement device may fail to detect an input of an injection button of the disposable insulin pen.

### SUMMARY OF THE INVENTION

An aspect of an embodiment of the present invention is to provide an injection button detection device having a function capable of detecting an injection button of an injector, to a dosage measurement device for an injector, capable of measuring a dosage.

Another aspect of an embodiment of the present invention is to provide an injector and injection button detection device capable of detecting an input of an injection button of the injector, with which the injection button detection device is combined, without direct electrical or mechanical contact.

In accordance with one aspect of the present invention, there is provided an injector. The injector includes an injection button; and a coil for radiating energy that informs an injection button detection device combined with the injector, of an input of the injection button.

In accordance with another aspect of the present invention, there is provided an injection button detection device. The injection button detection device includes a transmitter for radiating an electromagnetic wave associated with a specific voltage to radiate energy that informs an injector combined with the injection button detection device, of an input of an injection button; a receiver for receiving a voltage based on energy radiated from the injector; and a controller for detecting an input of the injection button based on the voltage received from the receiver.

### BRIEF DESCRIPTION OF THE DRAWINGS

The above and other aspects, features and advantages of certain embodiments of the present invention will be more apparent from the following description taken in conjunction with the accompanying drawings, in which:
FIG. 1 illustrates an injector and injection button detection device according to an embodiment of the present invention;
FIG. 2 illustrates a structure of an envelope detector shown in FIG. 1;
FIG. 3 illustrates voltage signals generated in an injector and injection button detection device for an injector according to an embodiment of the present invention;
FIGs. 4A and 4B illustrate detection of an envelope from a signal detected by an injection button detection device for an injector according to an embodiment of the present invention; and
FIGs. 5A and 5B illustrate a device for detecting an input strength of an injection button of an injector according to an embodiment of the present invention.

Throughout the drawings, the same drawing reference numerals will be understood to refer to the same elements, features and structures.

### DETAILED DESCRIPTION OF EMBODIMENTS OF THE PRESENT INVENTION

Embodiments of the present invention will now be described in detail with reference to the accompanying drawings. In the following description, specific details such as detailed configuration and components are merely provided to assist the overall understanding of the embodiments of the present invention. Therefore, it should be apparent to those skilled in the art that various changes and modifications of the embodiments described herein can be made without departing from the scope and spirit of the invention. In addition, descriptions of well-known functions and constructions are omitted for clarity and conciseness.

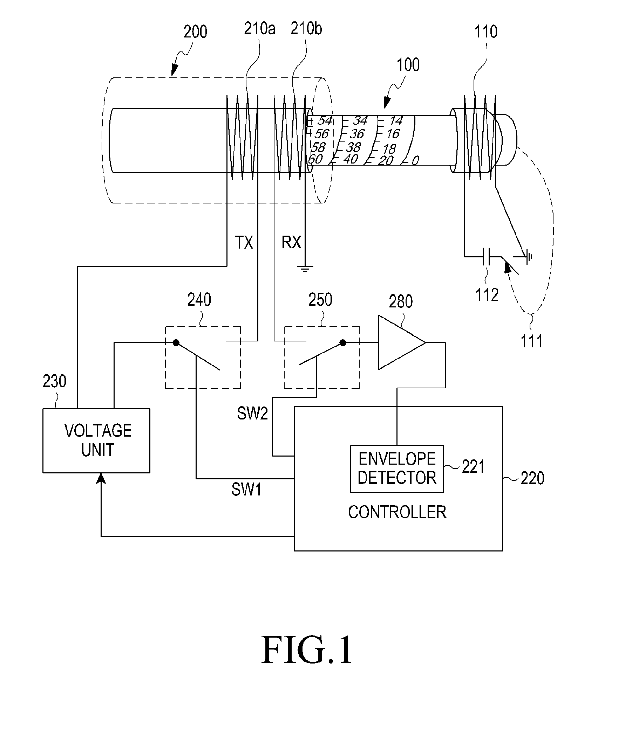

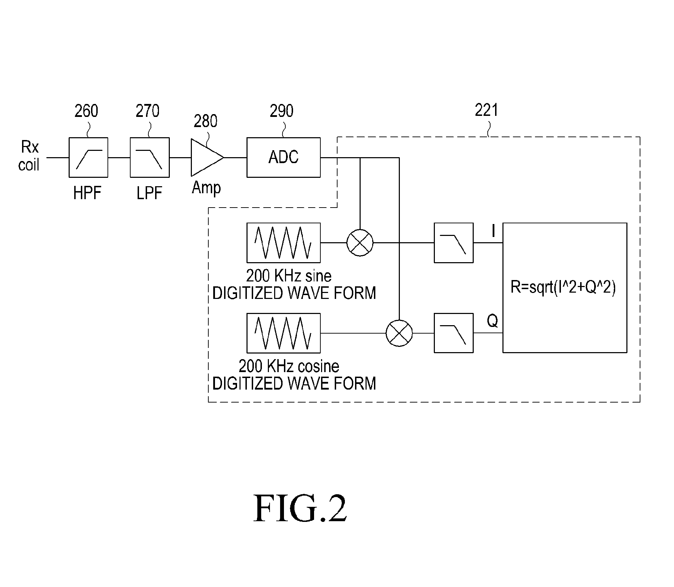

FIG. 1 illustrates an injector and injection button detection device according to an embodiment of the present invention. FIG. 2 illustrates a structure of an envelope detector shown in FIG. 1.

Referring to FIG. 1, the injection button detection device for an injector according to an embodiment of the present invention includes an injector 100 having a built-in coil that forms a resonant circuit when an injection button is input (pushed) to inject insulin, and an injection button detection device 200 that detects the input of the injection button of the injector 100 if a voltage occurs from the built-in coil in the resonant circuit formed in the injector 100 when the injection button detection device 200 is combined with the injector 100 to measure a dosage of the injector 100.

In structure, the injector 100 has an injection button 111 mounted on an end thereof, and at least one coil 110 and a capacitor 112 are installed in the injection button 111.

The at least one coil 110 is wound inside the injection button 111 and is connected to the injection button 111 and the capacitor 112.

The coil 110 and the capacitor 112 form a resonant circuit when the injection button 111 is input to inject insulin. When the resonant circuit is formed, electromagnetic waves radiated from the injection button detection device 200 store energy in the resonant circuit by electromagnetic induction, and the injector 100 radiates the energy stored in the resonant circuit through the at least one coil 110 mounted in the injection button 111.

The injection button detection device 200 includes a transmitter that radiates electromagnetic waves associated with a specific voltage in a transmitting-side coil based on the specific voltage generated at regular intervals, a receiver that receives a voltage generated in a receiving-side coil based on the energy radiated from the resonant frequency formed in the injector 100 after the generation of the electromagnetic waves radiated from the transmitter was completed, and a controller 220 for detecting an input of the injection button 111 based on the voltage received from the receiver.

The transmitter of the injection button detection device 200 includes a transmitting-side coil 210a, a voltage unit 230, and a first switch 240. The transmitting-side coil 210a is wound inside the injection button detection device 200 to radiate electromagnetic waves associated with a specific voltage received from the voltage unit 230. The voltage unit 230 provides the specific voltage to the transmitting-side coil 210a via the first switch 240 at regular intervals. The first switch 240 connects the voltage unit 230 to the transmitting-side coil 210a at regular intervals under control of the controller 220, to provide the specific voltage generated by the voltage unit 230 to the transmitting-side coil 210a.

The receiver of the injection button detection device 200 includes a receiving-side coil 210b and a second switch 250. The receiving-side coil 210b generates a voltage based on the energy radiated from the resonant frequency formed in the injector 100. The second switch 250, under control of the controller 220, connects the receiving-side coil 210b to the controller 220 to receive a voltage signal generated in the receiving-side coil 210b, if a predetermined time has elapsed after the generation of the electromagnetic waves radiated by the transmitter was completed.

As seen in FIG. 2, the receiver may include high and low pass filters 260 and 270 and an amplifier 280 for filtering and amplifying a voltage signal generated by the receiving-side coil 210b, and an Analog-to-Digital Converter (ADC) 290 for converting the analog voltage signal into a digital frequency signal. The second switch 250 connects the receiving-side coil 210b to the filters 260 and 270 under control of the controller 220. The received voltage signal is filtered and amplified by the filters 260 and 270 and the amplifier 280, converted into a digital frequency signal by the ADC 290, and provided to an envelope detector 221 of the controller 220. In an embodiment of the present invention, the second switch 250 is assumed to connect the receiving-side coil 210b to the filters 260 and 270.

The controller 220 of the injection button detection device 200 controls the first switch 240 to connect the transmitting-side coil 210a to the voltage unit 230 at regular intervals in the transmitter, and controls the second switch 250 to connect the receiving-side coil 210b to the filters 260 and 270 if a predetermined time has elapsed after generation of the electromagnetic waves radiated by the transmitter.

In an embodiment of the present invention, the transmitting-side coil 210a and the receiving-side coil 210b may be formed as a single coil.

When the transmitting-side coil 210a and the receiving-side coil 210b are formed as a single coil, the controller 220 connects the coil to the transmitter if the electromagnetic waves are generated, and connects the coil to the receiver after generation of the electromagnetic waves.

The controller 220 includes the envelope detector 221, as shown in FIG. 2, which detects an envelope signal from the voltage signal which was filtered and amplified by the filters 260 and 270 and the amplifier 280, and converted into a digital frequency signal by the ADC 290.

The envelope detector 221 detects an envelope from the received voltage signal by a digital lock-in detection method, and the controller 220 detects an input of the injection button 111 of the injector 100 when a level of the envelope signal detected by the envelope detector 221 is greater than or equal to a predetermined threshold.

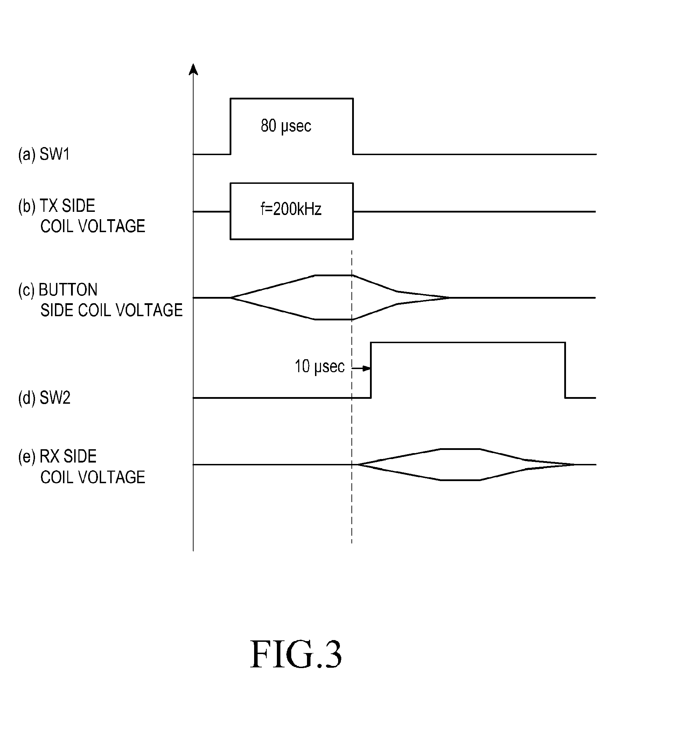

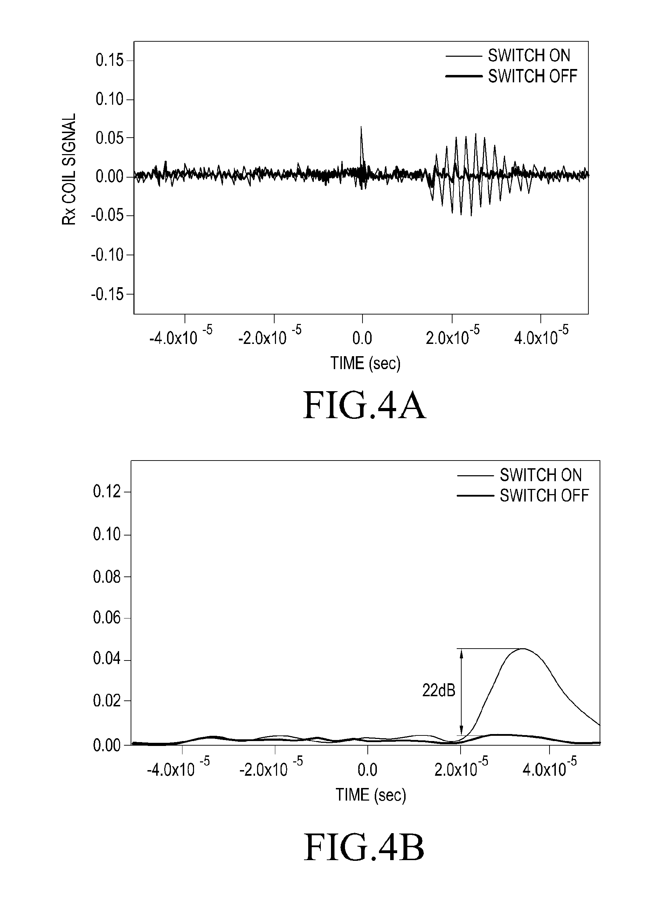

Detection of an input of an injection button of an injector by the injection button detection device for an injector illustrated in FIGs. 1 and 2 will now be described with reference to FIGs. 3 and 4.

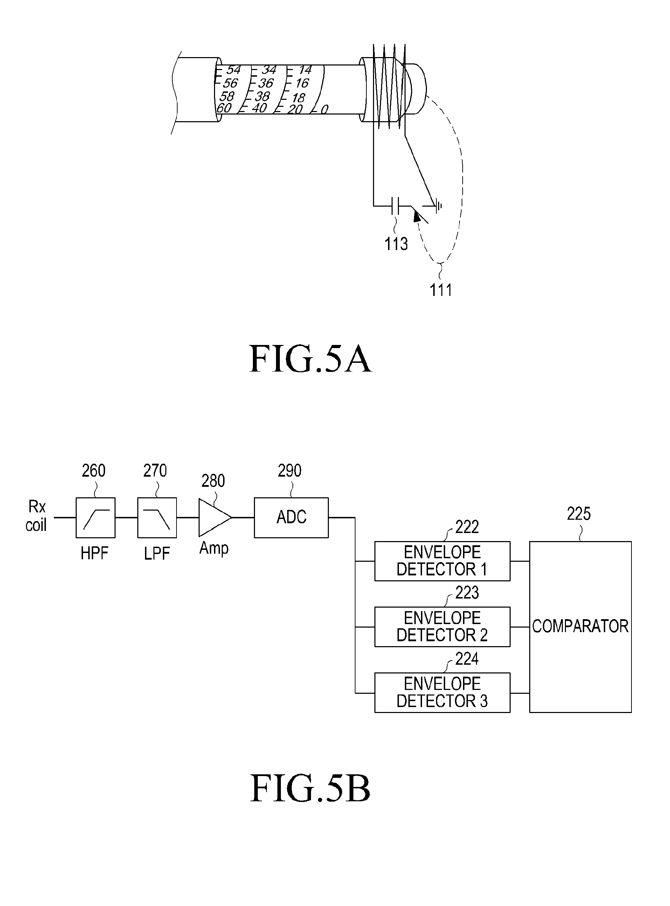

FIG. 3 illustrates voltage signals generated in an injector and injection button detection device according to an embodiment of the present invention. FIGs. 4A and 4B illustrate an envelope from a signal detected by an injection button detection device for an injector according to an embodiment of the present invention. FIGs. 5A and 5B illustrate a device for detecting an input strength of an injection button of an injector according to an embodiment of the present invention.

When the injection button detection device 200 is combined with the injector 100, if the controller 220 provides a first switch signal SW1 to the first switch 240, the first switch 240 is turned on, connecting the voltage unit 230 to the transmitting-side coil 210a. The voltage unit 230 connected to the transmitting-side coil 210a via the first switch 240 generates 200Khz square waves for 80 µsec, and 200Khz sine waves are reflected in the transmitting-side coil 210a by the generated voltage signal. In FIG. 3, waveforms A and B illustrate 200Khz square waves generated by the voltage unit 230 having an 80 µsec duration based on the first switch signal SW1.

If the injection button 111 of the injector 100 is input or pushed while the 200Khz electromagnetic square waves occur in the transmitting-side coil 210a, a resonant circuit is formed of the coil 110 and the capacitor 112 mounted in the injection button 111, and the electromagnetic waves generated in the transmitting-side coil 210a store energy in the formed resonant circuit by electromagnetic induction. The energy stored in the resonant circuit is radiated back in a specific voltage by the at least one coil 110 mounted in the injection button 111. Waveform C of FIG. 3 shows the voltage radiated by the at least one coil 110 due to the input of the injection button 111.

If 10 µsec have elapsed after generation of the electromagnetic waves associated with the voltage generated by the voltage unit 230, the controller 220 of the injection button detection device 200 provides a second switch signal SW2 to the second switch 250, connecting the receiving-side coil 210b to the filters 260 and 270.

If the receiving-side coil 210b is connected to the filters 260 and 270 via the second switch 250 while the voltage is radiated by the coil 110 in the injector 100, a voltage occurs in the receiving-side coil 210b by electromagnetic induction, and the controller 220 provides the generated voltage to the filters 260 and 270 to filter the voltage signal. Waveforms D and E of FIG. 3 illustrate the voltage generated by the receiving-side coil 210b received at the receiver of the injection button detection device 200, if 10 µsec have elapsed after generation of electromagnetic waves associated with the voltage generated by the voltage unit 230.

However, if the injection button 111 of the injector 100 is not input, the resonant circuit is not formed. In this state, even though the receiving-side coil 210b is connected to the filters 260 and 270 via the second switch 250, no signal is received at the filters 260 and 270 of the receiver.

The controller 220 filters and amplifies the voltage signal received from the receiving-side coil 210b by filters 260 and 270 and amplifier 280, and converts the analog voltage signals into a digital frequency signal in ADC 290.

FIG. 4A illustrates the voltage signal converted into the digital frequency signal by the ADC 290, and the controller 220 controls the envelope detector 221 to extract an envelope from the voltage signal shown in FIG. 4A.

The envelope detector 221 detects the envelope by the digital lock-in detection method. In operation, the envelope detector 221, which uses a sampling rate of 1M sample/sec, multiplies the digital frequency signal converted by the 12-bit ADC 290 by 200Khz sine wave values and 200Khz cosine wave values, which were numerically generated by the controller 220 in advance, generates I/Q signals by having the multiplied values undergo a 50Khz IIR(Infinite Impulse Response) digital low-pass filter, squares the I/Q signals, adds the results, and extracts only the envelope by square root.

The controller 220 performs detection of an envelope during the same period (80usec) as that of the 200Khz voltage provided by the voltage unit 230 to the transmitting-side coil 210a, and the controller 220 generates and stores, in units of 1M sample/sec, sine wave values and 200Khz cosine wave values in advance, whose phases have been shifted by 90° with respect to each other.

The extracted envelope has a carrier frequency of 200Khz and a width of about 100 µsec. By extracting only the envelope, the controller 220 may check the on/off-state for the injection button 111 of the injector 100. FIG. 4B illustrates the extracted envelope.

In an embodiment of the present invention, a coil may be wound inside the injection button detection device 200 in a tube to correspond to a cylindrical pen shape. A diameter of the injector 100 is about 16∼18 mm, and a diameter of the coil and a length of the tube may be adjusted to increase a Q value at a frequency of 200Khz, so as to improve the radiation and induction efficiency at the diameter. In the embodiment of the present invention, an enameled wire with a diameter of 0.18 mm was used, and a Q value was increased to 30 or more by setting the tube length as 11 mm. This coil has an L value of 60µH. The coil built in the injector 100 has the same structure as that of the coil provided in the injection button detection device 200, and may be connected to a 10nF capacitor so that it may resonate at the frequency of 200Khz, as used herein.

When combined with the injector 100, the injection button detection device 200 may detect an input of the injection button 111 of the injector 100, which is spaced apart from the injection button detection device 200 by about 35 mm.

The injection button detection device for an injector may detect not only an input of the injection button 111 of the injector 100, but also an input strength of the injection button 111.

FIGs. 5A and 5B illustrate a device for detecting an input strength of an injection button of an injector according to an embodiment of the present invention.

FIG. 5A illustrates a structure for detecting an input strength of the injection button 111 of the injector 100 in the injection button detection device 200, the injector 100 including a variable capacitor 113 whose capacitance varies depending on the input strength applied to the injection button 111.

As illustrated in FIG. 5B, the controller 220 in the injection button detection device 200 may include first to third envelope detectors 222 to 224, each having different reference frequencies, and a comparator 225 for comparing signal levels of envelopes detected by the first to third envelope detectors 222 to 224.

The variable capacitor 113 in the injector 100 may change the resonant frequency to correspond to the different reference frequencies, and the different reference frequencies of the first to third envelope detectors 222 to 224 may correspond to the reference frequencies of the digital lock-in detection method that is used to detect the envelopes.

Therefore, the input strength of the injection button 111 may be detected by applying, either in series or in parallel, the reference frequencies of the digital lock-in detection method as values in a frequency range, which may be changed depending on the input level of the injection button 111.

For example, when the reference frequencies of the first to third envelope detectors 222 to 224 are 200Khz, 180Khz, and 160Khz, respectively, the first to third envelope detectors 222 to 224 may extract envelopes for the voltages received from the receiving-side coil 210b by the digital lock-in detection method.

When the comparator 225 detects an envelope whose frequency signal has a highest level frequency from among the reference frequencies of the first to third envelope detectors 222 to 224, the controller 220 determines that an input strength corresponds to the reference frequency of the envelope detector that has detected the envelope having the greatest frequency signal.

Although generally the first envelope detector 222 having the reference frequency of 200Khz detects an envelope having the greatest frequency signal, occurrence of an error in the injector 100 may be detected if an envelope having the greatest frequency signal is detected by the third envelope detector 224 having the reference frequency of 160Khz.

Therefore, the present invention may detect not only the input strength of the injection button of the injector, but also the occurrence of an error in the injector.

As is apparent from the foregoing description, the injector and injection button detection device may detect an input of an injection button of an injector, with which the injection button detection device is combined, without direct electrical or mechanical contact.

While the invention has been shown and described with reference to certain embodiments thereof, it will be understood by those skilled in the art that various changes in form and details may be made therein without departing from the spirit and scope of the invention as defined by the appended claims and their equivalents.

## Claims

1. An injector comprising:
an injection button; and
a coil for radiating energy that informs an injection button detection device combined with the injector, of an input of the injection button.

2. The injector of claim 1, wherein when the injection button is input, a resonant circuit is formed by at least one coil and capacitor mounted in the injector button.

3. The injector of claim 2, wherein when the resonant circuit is formed, an electromagnetic wave radiated from the injection button detection device stores energy in the resonant circuit by electromagnetic induction, and the injector radiates the energy stored in the resonant circuit through the coil mounted in the injector to inform the injection button detection device of an input of the injection button.

4. The injector of claim 2, wherein the capacitor is a variable capacitor for varying a resonant frequency so that the injection button detection device combined with the injector may detect an input strength of the injection button.

5. An injection button detection device comprising:
a transmitter for radiating an electromagnetic wave associated with a specific voltage to radiate energy that informs an injector combined with the injection button detection device, of an input of an injection button;
a receiver for receiving a voltage based on energy radiated from the injector; and
a controller for detecting an input of the injection button based on the voltage received from the receiver.

6. The injection button detection device of claim 5, wherein the transmitter comprises:
at least one transmitting-side coil wound inside the injection button detection device to radiate the electromagnetic wave associated with the specific voltage;
a voltage unit for providing the specific voltage to the transmission-side coil at regular intervals; and
a first switch for connecting the voltage unit to the transmitting-side coil at regular intervals.

7. The injection button detection device of claim 5, wherein the receiver comprises :
at least one receiving-side coil for generating a voltage based on the energy radiated from the injector; and
a second switch for connecting the controller to the receiving-side coil after generation of the electromagnetic wave radiated by the transmitter.

8. The injection button detection device of claim 5, wherein the controller controls a first switch to connect the voltage unit to the transmitting-side coil in the transmitter at regular intervals, and controls a second switch to connect the controller to the receiving-side coil after generation of the electromagnetic wave radiated by the transmitter.

9. The injection button detection device of claim 5, wherein the controller includes an envelope detector for extracting an envelope from a voltage signal received from the receiver, and detecting an input of the injection button if a signal level of the extracted envelope is greater than or equal to a predetermined threshold.

10. The injection button detection device of claim 9, wherein the envelope detector detects the envelope by a digital lock-in detection method.

11. The injection button detection device of claim 5, wherein the injection button detection device is attachable and detachable to/from the injector.

12. The injection button detection device of claim 5, wherein the controller detects an input strength of the injection button based on the voltage received from the receiver.

13. The injection button detection device of claim 12, wherein the controller comprises :
a plurality of envelope detectors each having a different reference frequency and detecting an envelope of the received voltage signal; and
a comparator for comparing signal levels of the envelopes detected by the plurality of envelope detectors.

14. The injection button detection device of claim 13, wherein when the controller includes a plurality of envelope detectors, and a capacitor in the injector combined with the injection button detection device is a variable capacitor for varying a resonant frequency to correspond to a different reference frequency of each of the plurality of envelope detectors.

15. The injection button detection device of claim 13, wherein the different reference frequency of each of the plurality of envelope detectors is a reference frequency of a digital lock-in detection method used to detect the envelope.

16. The injection button detection device of claim 5, wherein when the transmitting-side coil in the transmitter and the receiving-side coil in the receiver are formed as a single coil, the coil is connected to the transmitter if the electromagnetic wave is generated, and the coil is connected to the receiver after generation of the electromagnetic wave is completed.
